# EUROPÄISCHE PATENTSCHRIFT

(11) **EP 1 317 187 B1**
(45) Veröffentlichungstag und Bekanntmachung des Hinweises auf die Patenterteilung: **07.12.2005**
(21) Anmeldenummer: 01977967.7
(22) Anmeldetag: 14.09.2001
(51) Int. Cl.: A23K 1/16, A23K 1/18

(54) **FUTTERMITTEL ODER FUTTERMITTELZUSATZ ALS LEISTUNGSFÖRDERER ODER APPETITFÖRDERER FÜR NUTZTIERE**
FEEDSTUFF OR A FEEDSTUFF ADDITIVE USED AS A PERFORMANCE PROMOTER OR AS AN APPETITE PROMOTER FOR DOMESTIC ANIMALS
ALIMENTS OU ADDITIF ALIMENTAIRE UTILISE COMME RENFOR ATEUR DE RENDEMENT OU COMME AGENT STIMULANT L'APPETIT POUR ANIMAUX

(30) Priorität: 15.09.2000 AT 15782000; 15.09.2000 AT 15792000
(43) Veröffentlichungstag der Anmeldung: 11.06.2003
(73) Patentinhaber: Roth, Hermann, 65343 Eltville (DE); Neufeld, Klaus, A-2532 Heiligenkreuz (AT)
(72) Erfinder: Roth, Hermann, 65343 Eltville (DE); Neufeld, Klaus, A-2532 Heiligenkreuz (AT)
(74) Vertreter: Puchberger, Rolf
(86) Internationale Anmeldenummer: PCT/AT2001/000291
(87) Internationale Veröffentlichungsnummer: WO 2002/021933

(56) Entgegenhaltungen:
- DE-A- 4 303 099
- DATABASE WPI Section Ch, Week 199519 Derwent Publications Ltd., London, GB; Class B04, AN 1995-145826 XP002191347 & SU 1 837 792 A (AS KIRG ORGANIC CHEM INST), 30. August 1993 (1993-08-30)
- DATABASE WPI Section Ch, Week 199906 Derwent Publications Ltd., London, GB; Class D13, AN 1999-061072 XP002191348 & CN 1 193 471 A (ZHAO H), 23. September 1998 (1998-09-23)
- CHEMICAL ABSTRACTS, vol. 101, no. 1, 2. Juli 1984 (1984-07-02) Columbus, Ohio, US; abstract no. 5919v, GUO, DAZHI ET AL.: "Effects of rapeseed meal, terramycin, berberine hydrochloride, and andrographolide on thyroid function in fattening swine" Seite 511; Spalte 1; XP002191345 & NUCL. TECH., Bd. 6, 1977, Seiten 67-68,
- CHEMICAL ABSTRACTS, vol. 118, no. 5, 1. Februar 1993 (1993-02-01) Columbus, Ohio, US; abstract no. 39240h, T. TAKESHITA ET AL.: "Isolation of berberine chloride as virucide for domestic animals" Seite 722; Spalte 1; XP002191346 & JP 04 139184 A (NIPPON MEKTRON CO., LTD.) 13. Mai 1992 (1992-05-13)
- DATABASE WPI Section Ch, Week 200143 Derwent Publications Ltd., London, GB; Class B04, AN 2001-398868 XP002191349 & CN 1 286 999 A (ZHOU L), 14. März 2001 (2001-03-14)

## Beschreibung

Die Erfindung betrifft ein Futtermittel als Leistungsförderer oder Appetitförderer für Nutztiere, wobei herkömmliche Futterstoffe wie Getreide oder Getreideprodukte, Mais, Proteine und aromatische Aminosäuren, Vitamine, mineralische Zuschlagstoffe wie Salze, Phosphate, Kalk, Enzyme und dergleichen enthalten sind, oder Futtermittelzusatz.

Sogenannte Leistungsförderer werden in der heutigen Nutztierfütterung üblicherweise angewendet. Man unterscheidet antibiotische und chemische Leistungsförderer wie etwa Zink-Bacitracin, Flavophospholipol, Virginiamycin, Tylosin-Phosphat, Avoparcin, Olaquindox und Monensin-Natrium, welche in Österreich als leistungsfördemde Futterzusatzustoffe zugelassen sind. Weiters gibt es selt einigen Jahren sogenannte Probiotica oder mikrobiologische Leistungsförderer.

Der Zweck dieser antibiotischen, chemischen und mikrobiologischen Leistungsförderer ist es, die Wachstumsintensität der Nutztiere zu erhöhen.

Chemische und antibiotische Leistungsförderer sind allerdings in den letzten Jahren, vor allem beim Konsumenten, in Verruf geraten und wurden etwa in Schweden als Putterzusatzstoffe verboten. Dies nicht so sehr wegen einer möglichen Rückstandsproblematik im Lebensmittel oder einer möglichen Gefährdung des Konsumenten, welche vielleicht durch früher zugelassene, in der Zwischenzeit jedoch verbotene Substanzen bestanden hat, sondern vielmehr durch den Wunsch des Konsumenten nach einem natürlichen, unverfälschten und gesundheitlich unbedenklichen Lebensmittel.

Die Entwicklung der mikrobiologischen Leistungsförderer hat diesem Wunsch teilweise Rechnung getragen, jedoch erfüllen diese nicht ganz die Erwartungen der modernen Landwirtschaft, die zu einem ökonomischen Produktionsprozess gezwungen ist.

Bekannt geworden ist aus der DE 43 03 099 A1 (DDr. Neufeld) die Verwendung von Benzophenanthridinalkaloiden zur Leistungsförderung. Diese Alkaloide, die insbesondere in der Sanguinaria Canadensis vorkommen, sind allerdings als natürliches Pflanzenmaterial nur in beschränkten Mengen erhältlich und somit teuer.

Die Verwendung von Berberin wird darin nicht erwähnt.

Aus der SU 1 837 792 (AS KIRG ORGANIC CHEM INST) ist die Verwendung eines Futtermittelzusatzes, der ein berberinhaltiges Material enthält, zur Leistungsförderung bei Nutztieren bekannt.

Aus der CN 1 193 471 (ZHAO H.) ist ein Futtermittelzusatz, der u.a. Berberin enthält, zur Wachstumsförderung bei Schweinen bekannt.

Aus GUO, DAZHI et al., "Effects of rapeseed meal, terramycin, berberine hydrochloride, and andrographolide on thyroid function in fattening swine", *Nucl. Tech.* 1983, (6), 67-8, 77, ist die Verbesserung der Gewichtszunahme bei Schweinen durch die Verabreichung von Berberin bekannt.

Aus der JP 04 139 184 (NIPPON MEKTRON CO., LTD.) ist die Verwendung von Berberinchlorid als Mittel gegen Darmerkrankungen bei Nutztieren wie Hühnern und Schweinen bekannt. Eine Verwendung des Berberinchlorids als Leistungsförderer ist darin nicht angeführt.

Überraschend hat sich gezeigt, dass die Leistungsförderung durch Protoberberinalkaloide und insbesondere durch den Einsatz einer Kombination von Benzophenanthridinalkaloiden und Protoberberinalkaloiden signifikant verbessert werden kann, wobei das diese Alkaloide enthaltende Pflanzenmaterial in größeren Mengen leichter erhalten werden kann.

Die Erfindung ist dadurch gekennzeichnet, dass ein Protoberberinalkaloid oder deren Derivate oder synthetische Analoge in Kombination mit einem Benzophenanthridinalkaloid oder deren Derivate oder synthetische Analoge in wirksamer Menge enthalten sind.

Weitere vorteilhafte Merkmale der Erfindung sind den Ansprüchen und der nachfolgenden Beschreibung zu entnehmen.

Die Protoberberinalkaloide können natürlichen Ursprungs oder synthetisch hergestellt sein. Ein natürlicher Ursprung sind Pflanzenbestandteile von Papaveraceae, Berberisarten, Coptisarten und auch die Rinde vom Phylodendron. Das bekannteste Alkaloid ist das Berberin. Beispielsweise sind aber auch andere Mitglieder dieser Gruppe wie Stylopin, Coptisin und Korisamin zu nennen.

Aus der Literatur ist zum Berberin bisher lediglich eine gewisse antimikrobielle Wirkung bekannt geworden. Die Schulmedizin kennt jedoch keine heilmittelmäßige Akzeptanz. Für die antimikrobielle Wirkung des Berberins sind relativ hohe Mengen des Alkaloids einzusetzen, was wirtschaftlich unrealistisch ist.

Wenn hier von Futtermittel die Rede ist, fallen unter die Erfindung ebenfalls alle entsprechenden Futterzusatzmittel und Vormischungen, die das Alkaloid enthalten und im Endeffekt die gleiche Wirkung ausüben, wie das fertige Futtermittel mit der entsprechenden Mischung.

Die Alkaloide können bevorzugt in Form der physiologisch verträglichem Salze vorgesehen werden, wie z.B. als Tannate, als Chloride oder als Sulfate.

Die bevorzugt einzusetzenden Mengen sind derart zu bemessen, dass die gewünschte Wirkung eintritt. Sie reicht von homöopathischen Dosierungen etwa bei 10⁻⁷ mg Alkaloid pro kg Futtermittel bis zu höheren Dosierungen von 50 mg Alkaloid pro kg Futtermittel. Ein bevorzugter Bereich liegt über 1mg Alkaloid pro Tonne fertiges Futtermittel, bei Vermischungen oder Futterzusätzen entsprechend höher.

Die Benzophenanthridinalkaloide kommen in der Natur beispielsweise in den Rhizomen der Kanadischen Blutwurzel Sanguinaria Canadensis vor, sowie in Wurzeln, Rhizoma und oberirdischen Pflanzenteilen von Chelidonium majus (Schöllkraut) und Macleaya cordata (Federmohn) vor. Diese Pflanzen zählen zu den Papaveraceae. Die Rhizome oder das Blattmaterial oder das gesamte Pflanzenmaterial können gesammelt und getrocknet werden. Es kann ein alkoholischer oder wässeriger Auszug gewonnen werden aus dem ein Pulver oder flüssige Formulierungen hergestellt werden. Bezogen auf die Trockensubstanz beträgt der Gehalt an Benzophenanthridinalkaloiden bis ca. 4 Gew.-%. Um Emteschwankungen auszusuchen, kann es vorteilhaft sein, den Gehalt des Hauptalkaloides Sanguinarin auf einen Wert von z.B. 1,5 Gew.-% durch Hinzugabe von. Füllstoffen einzustellen. Da etwa die Hälfte des natürlichen Alkaloldgehaltes auf das Sanguinarin entfällt, liegt somit der Gesamtalkaloidgehalt bei etwa 3 Gew.-%. Das Protoberberinalkaloid wird in der Natur z.B. in der Berberispflanze gefunden. Es kann als konzentrierter Extrakt gewonnen werden, oder als Pulver aus Pflanzenmaterial. Das Protoberberinalkaloid ist darüber hinaus in der Pflanze Chelidonium majus neben den Benzophenanthridinalkaloiden enthalten, wie auch in Coptisarten und der Rinde von Phylodendron.

Weitere Alkaloide der Benzophenanthridinalkaloide sind Chelerythrin, Chellrubin, Sanguirubin, Chelilutin und Sanguilutin.

Im Rahmen der Erfindung kann es auch vorteilhaft sein, Extrakte des Pflanzenmateriales oder die isolierten Alkaloide oder Protoberberinalkaloide und deren Derivate oder deren synthetische Analoge zu verabreichen.

Die Literatur nennt zum Schöllkraut (Chelidonium majus) z.B. folgende Kombinationen von Alkaloiden:

| Alkalold | Wurzel | Rhizom | Blätter |
|---|---|---|---|
| Coptisin | 0,33 | 0,59 | 1,07 |
| Chelidonin | 1,14 | 1,28 | 0,07 |
| Berberin | 0,07 | 0,07 | 0,11 |
| Chelerythrin | 0,77 | 1,08 | 0,04 |
| Sanguinarin | 0,14 | 0,37 | 0,01 |
| Summe | 2,45 | 3,37 | 1,30 |

Die im Futter enthaltene Alkaloidmenge und Protoberberinalkaloidmenge ist nach unten lediglich durch die Wirksamkeit begrenzt. Die Gesamtalkaloidmenge pro Tonne Futtermittel ist bevorzugt größer 1 mg. Bezogen auf das standardisierte getrocknete Papaveraceae-Pflanzenmaterial entspricht dies etwa 0,067 g je Tonne Futtermittel.

In einer genauer untersuchten Futtermittelverabreichung wurden Mengen zwischen 5 g und 150 g getrockneten Papaveraceae Pflanzenmaterial je t Futter verabreicht. Das Pflanzenmaterial war auf 1,5 % Alkaloid Sanguinarin standardisiert, wobei der Gesamtalkaloidgehalt ca. 3 % betrug. Zusätzlich wurde Berberin in einer Konzentration von 0,1 bis 25 g je Tonne Futter verabreicht.

Die gegenständliche Erfindung umfasst auch Futterzusatzmittel wie z.B. Vormischungen (Premixe) oder Mineralfutter oder Ergänzungsfuttermittel zur Zubereitung von Futtermittel, wobei das Zusatzmittel neben den üblichen Futterzusatzstoffen die Alkaloide oder deren Derivate und Analoge oder diese Alkaloide enthaltende Pflanzenbestandteile der Papaveraceae enthält. Somit umfasst die Erfindung sowohl das an das Tier verfütterte Futter selbst, als auch die Vorprodukte, die vom Anwender dazu benutzt werden, das fertige Futtermittel herzustellen.

Im folgenden wird die Erfindung an Hand von Beispielen näher erläutert.

### 1. Beispiel einer herkömmlichen Futtermittelrezeptur für Hühner und Schweine:

| | (in Gewichtsprozent) |
|---|---|
| Weizen: | 42 % |
| Mais: | 20 % |
| Sonnenblumen: | 3,6 % |
| Soyaschrot I | 4 % |
| Soyaschrot II | 16,6 % |
| Rapsschrot | 4 % |
| Futteröl | 6,5 % |
| Methionin | 0,26 % |
| Lysin | 0,48 % |
| Mono Calcium Phosphat | 0,85 % |
| Kalk | 0,5 % |
| Salz | 0,025 % |
| Vormischung | 1,0 % |
| Enzyme | 0,2 % |

| Aminosäuren: | |
|---|---|
| Protein | 19,2 % |
| Lysin | 0,824 % |
| Methionin | 0,276 % |
| Meth + Cystin | 0,568 % |
| Threonin: | 0,8 % |
| Thryptophan | 0,24 % |

### 2. Beispiel gemäß Erfindung:

Das Futtermittel für Schweine entspricht Beispiel 1. Die angegebenen Mengen zugesetzte Papaveraceae sind in g getrocknetes Wurzelmaterial mit 1,5 Gew.-% Alkaloid Sanguinarin pro Tonne Futtermittel. Tierrasse: Great Yorkshire + Kreuzungssauen, pro Gruppe 23-24 Tiere.

| Gruppen untersuchte Tiere: | | a | b | c |
|---|---|---|---|---|
| Papaveraceae g/t | Starter/Vormast 22kg - 48 kg | 0 | 0 | 15 |
| | Endmast 48 kg - 109 kg | 0 | 30 | 30 |

### Resultat:

### Klassifizierung und Schlachtkörperwert

(AA besser als A besser als B):

| | 0-Kontrolle | Papaveraceae-Dosierungen (Vormast/Endmast) | |
|---|---|---|---|
| | (0/0) | (0/30) | (15/30) |
| Magerfleisch % | 54.5 | 55.1 | 55.0 NS |
| Rückenspeck mm | 17.8 | 17.7 | 17.6 NS |
| Muskelfläche | 51.9 | 55.6 | 54.2 (p <0.05) |
| Klasse AA + A % | 77% | 96% | 92% |
| Klasse B | 23 % | 4 % | 8 % |
| Tageszunahme | 100 | 102 | 108 |
| Futteraufnahme | 100 | 90 | 92 |

Somit ist gemäß der Erfindung der Anteil der Klasse AA+A signifikant erhöht.

### 3. Beispiel:

| | | |
|---|---|---|
| Gruppen | O-Kontrolle | 30g Papaveraceae/t Futtermittel |
| | 144 Ferkel am Start, 72 je Gruppe, verteilt auf 8 Wiederholungen je Gruppe. Restriktive Fütterung. | |

### Vormast-Endmast 31-115 kg:

| | 0-Kontrolle | BA Alkaloide + Berberin/Coptisin 30g/t |
|---|---|---|
| Magerfleisch (FOM)% | 54.9 | 55.2 |
| Muskelfläche cm² | 51.7 | 52.0 |
| Rückenspeck cm | 20.6 | 20.1 |
| Tageszunahme | 100 | 105 |
| Futteraufnahme | 100 | 94,6 |

Wirtschaftlichkeit: Basis 1.022 Euro/kg Schlachtgewicht

| | |
|---|---|
| Schlachtkörperwert Euro | +1.53 |
| Papaveraceae Vorteil Euro | +2.71 |
| (BA Alkaloide = Benzophenantridinalkaloide) | |

### Kommentar:

In einem System mit restriktiver Fütterung erhöht Papaveraceae den Schlachtkörperwert, indem der Anteil an Magerfleisch und Muskelfläche steigt und zugleich der Rückenspeckwert sinkt. Dadurch erhöht Papaveraceae die Fleischqualität und die Rentabilität um ca. 1,53 Euro je Tier. Die Tageszunahme steigt um 5 % und der Futteraufwand sinkt um 5,4 %. Der zusätzliche Vorteil liegt bei 1,5 EURO.

### 4. Beispiel:

In einem Versuch der Mastprüfungsanstalt der BLT Grub (Deutschland) an Geflügel (Brollermast) wurden folgende Ergebnisse erzielt.

| | Kontrolle | BA Alkaloide 2 mg/kg Futter | Berberin/Coptisin 25 mg/kg Futter | BA Alkaloide +Berberin/Coptisin 2 mg/kg Futter |
|---|---|---|---|---|
| Zunahmen | 100 | 98 | 100 | 102 |
| Futterverbrauch | 100 | 95 | 98 | 100 |
| Futterverwertung | 100 | 97 | 98 | 98 |
| Mastkennzahl | 100 | 102 | 102 | 104 |
| Europ. Broiler-Index | 312 | 317 | 317 | 323 |

Schon Berberin/Coptisin zeigt eine Mastkennzahl vergleichbar mit jener von Benzophenanthridinalkaloid. Die Kombination Benzophenanthridinalkaloide mit Protoberberinalkaloiden (Berberin/Coptisin) zeigt eine signifikante Verbesserung der Mastkennzahl mit 104 gegenüber 102 bzw. 100.

Die Broilermast mit verschiedenen erfindungsgemäßen Konzeptionen auf Basis von Papaveraceae Wurzeimaterial (Sanguinaria canadensis und Chelidonium majus) sowie Berberin mit den Wirkstoffen der Benzophenanthridinalkaloide, Protoberberin und Coptisin zeigte signifikant positive Einflüsse in einem Futter mit 60 ppm Salinomycin:

| | |
|---|---|
| signifikant höheres Endgewicht | bis +7,3 % |
| signifikant höheres Schlachtgewicht | bis +7,1 % |
| signifikant reduzierter Futteraufwand: 0,03 bis 0,05 Punkte: | bis -3% |
| keine Veränderung am Anteil wertvoller Teilstücke deutlich signifikant reduzierter Wasserverbrauch | bis -10% |
| signifikant verbesserte Mastkennzahl | bis +11 Punkte |

## Patentansprüche

1. Futtermittel als Leistungsförderer oder Appetitförderer für Nutztiere, wobei herkömmliche. Futterstoffe wie Getreide oder Getreideprodukte, Mais, Proteine und aromatische Aminosäuren, Vitamine, mineralische Zuschlagstoffe wie Salze, Phosphate, Kalk, Enzyme und dergleichen enthalten sind, oder Futtermitteizusatz, **dadurch gekennzeichnet, dass** ein Protoberberinalkaloid oder deren Derivate oder synthetische Analoge in Kombination mit einem Benzophenanthridinalkaloid oder deren Derivaten oder synthetischen Analogen in wirksamer Menge enthalten ist.

2. Futtermittel oder Futtermittelzusatz nach Anspruch 1, **dadurch gekennzeichnet, dass** die Alkaloidmenge pro t Futtermittel größer als 1 mg ist.

3. Futtermittel oder Futtermittelzusatz nach Anspruch 1 oder 2, **dadurch gekennzeichnet, dass** die Alkaloide in Form von die Alkaloide enthaltendem Pflanzenmaterial oder als Extrakt davon in Form der isolierten Alkaloide oder Alkaloidgemische oder synthetische Analoge vorliegen.

4. Futtermittel oder Futtermittelzusatz nach einem der Ansprüche 1 bis 3, **dadurch gekennzeichnet, dass** als Pflanzenmaterial Wurzeln und/oder Rhizomen und/oder oberirdische Pflanzenteile der Papaveraceae oder anderer Protoberberinalkaloide enthaltender Pflanzen enthalten sind.

5. Futtermittel oder Futtermittelzusatz nach Anspruch 4, **dadurch gekennzeichnet, dass** Pflanzenmaterial aus der Gruppe *Sanguinaria canadensis, Cheildonium majus, Macleaya cordata*, Berberispflanze, Coptisarten und Phylodendron-Rindenmaterial ausgewählt ist

6. Verwendung von Protoberberinalkaloiden oder deren Derivaten oder synthetischen Analogen in Kombination mit Benzophenanthridinalkaloiden oder deren Derivaten oder synthetischen Analogen oder von diese Alkaloide enthaltendem Pflanzenmaterial zur Leistungsförderung für Nutztiere.

## Claims

1. A feedstuff as a performance promoter or appetite promoter for livestock, containing conventional feedstuffs such as cereal or cereal products, maize, proteins and aromatic amino acids, vitamins, inorganic additives such as salts, phosphates, lime, enzymes and the like, or a feed additive, **characterised in that** it contains an effective quantity of a protoberberine alkaloid or derivatives or synthetic analogues thereof in combination with a benzophenanthridine alkaloid or derivatives or synthetic analogues thereof.

2. A feedstuff or feed additive according to claim 1, **characterised in that** the quantity of alkaloid per tonne of feedstuff is greater than 1 mg.

3. A feedstuff or feed additive according to claim 1 or claim 2, **characterised in that** the alkaloids are present in the form of plant material containing the alkaloids or as an extract thereof in the form of the isolated alkaloids or alkaloid mixtures or synthetic analogues.

4. A feedstuff or feed additive according to any one of claims 1 to 3, **characterised in that** the plant material present comprises roots and/or rhizomes and/or above-ground plant parts of Papaveraceae or other plants containing protoberberine alkaloids.

5. A feedstuff or feed additive according to claim 4, **characterised in that** plant material is selected from the group *Sanguinaria canadensis, Chelidonium majus*, *Macleaya cordata*, Berberis plants, Coptis species and philodendron bark material.

6. Use of protoberberine alkaloids or the derivatives or synthetic analogues thereof in combination with benzophenanthridine alkaloids or the derivatives or synthetic analogues thereof or of plant material containing these alkaloids for promoting livestock performance.

## Revendications

1. Aliment favorisant le développement ou l'appétit des animaux, dans lequel figurent des substances alimentaires habituelles, comme des céréales ou des produits céréaliers, du maïs, des protéines et des acides aminés aromatiques, des vitamines, des additifs minéraux comme des sels, des phosphates, de la chaux, des enzymes et des produits similaires, ou additif alimentaire, **caractérisé en ce qu'**il contient un alcaloïde protoberbérine ou ses dérivés ou des analogues synthétiques, en combinaison avec un alcaloïde benzophénanthridine ou ses dérivés ou des analogues synthétiques, en une quantité efficace.

2. Aliment ou additif alimentaire selon la revendication 1, **caractérisé en ce que** la quantité d'alcaloïdes est supérieure à 1 mg par tonne d'aliment.

3. Aliment ou additif alimentaire selon la revendication 1 ou 2, **caractérisé en ce que** les alcaloïdes sont présents sous la forme de matières végétales contenant les alcaloïdes ou comme extrait de ces matières végétales, sous la forme des alcaloïdes isolés ou de mélange d'alcaloïdes ou d'analogues synthétiques.

4. Aliment ou additif alimentaire selon l'une quelconque des revendications 1 à 3, **caractérisé en ce que** les matières végétales englobent les racines et/ou les rhizomes et/ou les parties végétales aériennnes des papavéracées ou d'autres plantes contenant des alcaloïdes protoberbérine.

5. Aliment ou additif alimentaire selon la revendication 4, **caractérisé en ce que** les matières végétales sont choisies dans le groupe formé par Sanguinaria canadensis, Chelidonium majus, Macleaya cordata, le Berbéris, les espèces de Coptis et les écorces de Phylodendron.

6. Utilisation d'alcaloïdes protoberbérine ou de leurs dérivés ou d'analogues synthétiques, en combinaison avec des alcaloïdes benzophénanthridine ou leurs dérivés ou des analogues synthétiques, ou de matières végétales contenant ces alcaloïdes, pour faciliter le développement des animaux.
